(19) Europäisches Patentamt
European Patent Office
Office européen des brevets

(11) EP 4 789 661 A1

(12) EUROPEAN PATENT APPLICATION

(43) Date of publication:
**12.08.2026 Bulletin 2026/33**

(21) Application number: **25156471.2**

(22) Date of filing: **07.02.2025**

(51) International Patent Classification (IPC):
***A61K 9/28*** *(2006.01)* ***A61K 31/135*** *(2006.01)*
***A61K 31/167*** *(2006.01)* ***A61K 31/192*** *(2006.01)*
***A61K 31/522*** *(2006.01)*

(52) Cooperative Patent Classification (CPC):
(C-Sets available)
**A61K 9/282; A61K 31/167; A61K 31/192; A61K 31/522** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Haleon CH SARL**
**1197 Prangins (CH)**

(72) Inventor: **The designation of the inventor has not yet been filed**

(74) Representative: **Haleon Patent Department**
**Building 5, First Floor**
**The Heights**
**Weybridge, Surrey KT13 0NY (GB)**

# (54) COMPRESSED PHARMACEUTICAL DOSAGE FORM

(57) The present invention relates to compressed pharmaceutical dosage forms for oral administration and methods of manufacturing. Particularly, the pharmaceutical dosage form is a coated tablet that has a coating consisting of wax alone.

EP 4 789 661 A1

(52) Cooperative Patent Classification (CPC): (Cont.)

C-Sets
**A61K 31/167, A61K 2300/00;**
**A61K 31/192, A61K 2300/00;**
**A61K 31/522, A61K 2300/00**

## Description

### Field of the Invention

The present invention relates to compressed pharmaceutical dosage forms for oral administration and methods of manufacturing. Particularly, the pharmaceutical dosage form is a coated tablet that has a coating consisting of wax alone.

### Background of the Invention

Over the counter (OTC) medications are frequently marketed as oral tablets. To ensure acceptability and patient comfort, the tablets need to be easy to swallow, without any unpleasant taste. In general, this is achieved via the application of a suitable coating at the end of the manufacturing process.

The coating is generally applied in a coating pan after the actual tableting process has been completed. Coating solution is sprayed on the uncoated tablets, and the tablets are rotated in the coating pan with heated air being blown on the tablets to dry. Film coatings based on an aqueous solution comprising a polymer are most frequently used, but sugar-based coatings are also common. Such coatings can be used to protect the product from light, moisture and oxygen and may also affect the location or rate of release of an active ingredient. Coatings are frequently also coloured, which allows patients to distinguish different tablets they may be taking and to thereby avoid any dosing mistakes. Sometimes, at the end of the coating process, a wax polishing step may be performed to provide an aesthetically pleasing, shiny finish to the product.

However, the coating process has a number of downsides. For example, many coatings used in the pharmaceutical industry contain ingredients such as titanium dioxide that may be considered problematic in some territories. In addition, the coating process typically takes several hours, requiring large amounts of energy, which increases manufacturing costs. Further, the use of rotating drums for extended periods of time, increases the risk of damage or breaking of tablets, leading to wastage and necessitating additional processing steps.

Thus, there is a need for improvements that overcome the disadvantages described above.

### Summary of the Invention

In a first aspect of the invention, there is provided a compressed pharmaceutical dosage form for oral administration comprising at least one active pharmaceutical ingredient (API) and one or more pharmaceutically acceptable diluents, excipients or carriers, wherein the pharmaceutical dosage form is a coated tablet, wherein the coating consists of wax and wherein the total weight of wax is from about 0.01% w/w to about 0.5% w/w based on the weight of the tablet core.

Particularly, the at least one API is selected from the group consisting of diclofenac, ketoprofen, naproxen, sodium salicylate, acetylsalicylic acid, ibuprofen, dexibuprofen, paracetamol (acetaminophen), and combinations thereof.

Preferably the at least one API is selected from the group consisting of paracetamol, ibuprofen, dexibuprofen and combinations thereof.

Particularly, the compressed pharmaceutical dosage form may comprise from 200mg to 1000mg of paracetamol and/or from 80mg to 400mg of ibuprofen or equivalent ibuprofen salt. Thus, in certain embodiments, the compressed pharmaceutical dosage form may comprise from 200mg to 1000mg of paracetamol. In certain embodiments, the compressed pharmaceutical dosage form may comprise from 80mg to 400mg of ibuprofen or equivalent ibuprofen salt. In certain embodiments, the compressed pharmaceutical dosage form may comprise from 200mg to 1000mg of paracetamol and from 80mg to 400mg of ibuprofen or equivalent ibuprofen salt.

Particularly, the compressed pharmaceutical dosage form may comprise from 200mg to 1000mg of paracetamol and/or from 40mg to 200mg of dexibuprofen or equivalent dexibuprofen salt. Thus, in certain embodiments, the compressed pharmaceutical dosage form may comprise from 40mg to 200mg of dexibuprofen or equivalent dexibuprofen salt. In certain embodiments, the compressed pharmaceutical dosage form may comprise from 200mg to 1000mg of paracetamol and from 40mg to 200mg of dexibuprofen or equivalent dexibuprofen salt.

The compressed pharmaceutical dosage form may comprise from 125mg to 500mg of naproxen or equivalent naproxen salt. Thus, in certain embodiments, the compressed pharmaceutical dosage form may comprise from 125mg to 500mg of naproxen sodium.

The compressed pharmaceutical dosage form may comprise from 25mg to 100mg of diclofenac or equivalent diclofenac salt. Thus, in certain embodiments, the compressed pharmaceutical dosage form may comprise from 25mg to 100mg of diclofenac sodium.

The compressed pharmaceutical dosage form may comprise 500mg of paracetamol and 25mg of diphenhydramine chloride.

Particularly, the wax is selected from the group consisting of carnauba wax, candelilla wax, beeswax (white or yellow), hard paraffin wax, cetyl palmitate, rice bran wax, and combinations thereof. Preferably, the wax is carnauba wax.

In a Second Aspect of the Invention, there is provided a method of manufacturing the compressed pharmaceutical dosage form of the First Aspect.

Particularly, the method for manufacturing the compressed pharmaceutical dosage form comprises the steps of preparing a tablet core and directly coating the outer surface of the tablet core with a coating consisting of wax. Particularly, the total weight of wax is from about 0.01% w/w to about 0.5% w/w based on the weight of the tablet core. Preferably, the wax is carnauba wax.

Particularly, the step of directly coating the outer surface of the tablet core with a coating consisting of wax may comprise, transferring a tablet core into a coating pan, adding a wax powder to the coating pan, heating and rotating the coating pan.

Particularly, the step of directly coating the outer surface of the tablet core with a coating consisting of wax may comprise spraying molten wax onto the tablets.

The method for manufacturing may further comprise the step of packaging the coated tablets.

## Brief description of Figures

**Figure 1:** Experimental set-up for a comparative slide test to compare a commercial product with a product according to the invention; (1) stainless-steel ramp, (2) ramp angle, (3) slide lane, (4) paper ribbon, slide borders, (5) test tablet.

**Figure 2:** Results of a comparative slide test comparing a commercial product with a product according to the invention.

## Description of the Invention

The present Inventors have identified a need to develop more sustainable means for producing coated tablets, reducing the cost, complexity and energy required. Surprisingly, the use of a coating consisting of wax reduces the complexity of the production process, significantly decreasing the energy and resources used, thereby increasing sustainability whilst also retaining many of the benefits of existing coated pharmaceutical dosage forms.

There is therefore provided, a compressed pharmaceutical dosage form, specifically a coated tablet for oral administration having a coating that consists of wax, the total weight of the wax being from about 0.02% w/w to about 0.5% w/w based on the weight of the tablet core. Compressed pharmaceutical compositions of the invention comprise a compressed core (e.g. a tablet core) and a coating consisting of wax.

Pharmaceutical dosage forms are dosage forms comprising a safe and effective amount of at least one active ingredient and pharmaceutically acceptable excipients in final form suitable for administration to a patient. The dosage forms are for oral administration, via the mouth, intended to be delivered to or released in the stomach or small intestine of a patient.

The terms "compressed core" and "tablet core" refer to a non-liquid oral dosage form intended to be swallowed and having a sufficiently defined form to be coated. Preferably the composition of the tablet core is a homogenous composition in which at least one active pharmaceutical ingredient (API) and one or more pharmaceutically acceptable diluents, excipients or carriers are blended together to form a homogenous solid core with a uniform structure or composition throughout, free of discreet zones or layers of the active agent combinations. The homogenous solid core is formed by compression methods known in the art using conventional equipment and processes, for example by compressing uniform volumes of powder, particles or particle aggregates produced by granulation methods.

The tablet core may be of any size or shape known in the art, such as, by way of non-limiting example round, elliptical, oval, square or triangular.

The compressed pharmaceutical dosage form, may comprise one or more debossed patterns, applied to the tablet core, such as, by way of non-limiting example, at least one letter and/or at least one number. Surprisingly, the use of a wax coating consisting of about 0.02% w/w to about 0.5% w/w of wax based on the weight of the tablet core, does not fill the debossed pattern, retaining the *'crispness'* of the edges of the pattern and enabling, for example, tablet identification without the need for ink.

The tablet core is directly coated with a coating consisting of wax, the total weight of the wax being from about 0.01% w/w to about 0.5% w/w based on the weight of the tablet core. For example, from 0.01% w/w to 0.5% w/w, from 0.01% w/w to 0.45% w/w, from 0.01% w/w to 0.40% w/w, from 0.01% w/w to 0.35% w/w, from 0.01% w/w to 0.30% w/w, from 0.01% w/w to 0.25% w/w, from 0.01% w/w to 0.20% w/w, from 0.01% w/w to 0.15% w/w or from 0.01% w/w to 0.10% w/w. For example, from 0.02% w/w to 0.5% w/w, from 0.02% w/w to 0.45% w/w, from 0.02% w/w to 0.40% w/w, from 0.02% w/w

to 0.35% w/w, from 0.02% w/w to 0.30% w/w, from 0.02% w/w to 0.25% w/w, from 0.02% w/w to 0.20% w/w, from 0.02% w/w to 0.15% w/w or from 0.02% w/w to 0.10% w/w. For example, about 0.01% w/w, 0.02% w/w, 0.03% w/w, 0.04% w/w, 0.05% w/w, 0.06% w/w, 0.07% w/w, 0.08% w/w, 0.09% w/w, 0.10% w/w, 0.15% w/w, 0.20% w/w, 0.25% w/w, 0.30% w/w, 0.35% w/w, 0.40% w/w, 0.45% w/w or 0.50% w/w. In some embodiments, approximately 0.079kg of wax is used per 400kg of tablet cores.

The term "coating" refers to a substantially continuous layer surrounding the core of the compressed pharmaceutical composition. The term "directly coated" refers to a configuration whereby the layer of wax is applied to the surface of the compressed core with no intervening layers or structures present between the compressed core and the coating layer. The layer of wax is the only coating applied. Thus, the wax coating directly contacts the surface of the compressed core and forms the outer surface of the dosage form that is exposed to the atmosphere or external environment, i.e. it is a single, monolayer coating of wax with no additional material added beyond the wax.

The compressed pharmaceutical dosage forms comprise at least one active pharmaceutical ingredient (API). An "active pharmaceutical ingredient" is a term used in the art to refer to a drug substance or mixture of drug substances intended to be used in the manufacture of a drug product and that, when used in the production of a drug, becomes an active ingredient in the drug product. Such substances have pharmacological activity within the body of a patient.

Particularly, the at least one API is useful for treating a variety of types of pain. The term "pain" encompasses both acute and chronic pain. Acute pain means immediate, generally high threshold, pain brought about by physical injury such as a cut, crush or burn for example. Chronic pain is pain other than acute pain and includes, without limitation, neuropathic pain, visceral pain, inflammatory pain, headache pain, muscle pain and referred pain. Chronic pain may be of relatively long duration, for example, months or years and can be continuous or it may be intermittent, such as headache pain. Particular types of pain include headache pain, menstrual pain, dental pain, jaw pain and arthritis pain.

Particularly, the at least one API is selected from the group consisting of diclofenac, ketoprofen, naproxen, sodium salicylate, acetylsalicylic acid, ibuprofen, dexibuprofen, paracetamol (acetaminophen), and combinations thereof.

It should be understood that reference to diclofenac, ibuprofen and dexibuprofen herein is also intended to include their pharmaceutically acceptable salts, for example, diclofenac sodium, ibuprofen lysine, naproxen sodium and the like.

Preferably the at least one API is selected from the group consisting of paracetamol, ibuprofen, dexibuprofen and combinations thereof.

Particularly, the compressed pharmaceutical dosage form may comprise from 200mg to 1000mg of paracetamol and/or from 80mg to 400mg of ibuprofen or equivalent ibuprofen salt. Thus, in certain embodiments, the compressed pharmaceutical dosage form may comprise from 200mg to 1000mg of paracetamol. In certain embodiments, the compressed pharmaceutical dosage form may comprise from 80mg to 400mg of ibuprofen or equivalent ibuprofen salt. In certain embodiments, the compressed pharmaceutical dosage form may comprise from 200mg to 1000mg of paracetamol and from 80mg to 400mg of ibuprofen or equivalent ibuprofen salt.

Particularly, the compressed pharmaceutical dosage form may comprise from 200mg to 1000mg of paracetamol and/or from 40mg to 200mg of dexibuprofen or equivalent dexibuprofen salt. Thus, in certain embodiments, the compressed pharmaceutical dosage form may comprise from 40mg to 200mg of dexibuprofen or equivalent dexibuprofen salt. In certain embodiments, the compressed pharmaceutical dosage form may comprise from 200mg to 1000mg of paracetamol and from 40mg to 200mg of dexibuprofen or equivalent dexibuprofen salt.

The compressed pharmaceutical dosage form may comprise from 125mg to 500mg of naproxen or equivalent naproxen salt.

The compressed pharmaceutical dosage form may comprise from 25mg to 100mg of diclofenac or equivalent diclofenac salt.

In certain embodiments, the at least one API may optionally be combined with caffeine, diphenhydramine chloride, phenylephrine HCl or pseudoephedrine.

The compressed pharmaceutical dosage form may comprise 500mg of paracetamol and 25mg of diphenhydramine chloride.

Compressed pharmaceutical dosage forms of the Invention comprise one or more pharmaceutically acceptable diluents, excipients or carriers including, but not limited to, polymers, resins, plasticizers, fillers, lubricants, binders, disintegrants, solvents, cosolvents, surfactants, preservatives, sweetener agents, flavouring agents, buffer systems, pharmaceutical-grade dyes and pigments.

Preferably, the wax is selected from the group consisting of carnauba wax, candelilla wax, beeswax (white or yellow), hard paraffin wax, cetyl palmitate, rice bran wax, and combinations thereof. More preferably, the wax is carnauba wax. In some embodiments, the wax may be a coloured wax, for example, coloured with a colouring agent.

There is also provided a method of manufacturing the compressed pharmaceutical dosage form. Particularly, the method for manufacturing the compressed pharmaceutical dosage form comprises the steps of preparing an uncoated tablet core and directly coating the outer surface of the tablet core with a coating consisting of wax.

Particularly, the method for manufacturing the compressed pharmaceutical dosage form comprises the steps of

preparing an uncoated tablet core and directly coating the outer surface of the tablet core with a coating consisting of wax.

Particularly, the method for manufacturing the compressed pharmaceutical dosage form comprises the steps of preparing an uncoated tablet core and a coating step consisting of directly coating the outer surface of the tablet core with a coating consisting of wax.

Particularly, the total weight of wax is from about 0.01% w/w to about 0.5% w/w based on the weight of the tablet core. Preferably, the wax is carnauba wax. Particularly, about 0.079kg of wax is used per 400kg of tablet cores.

Coating can be achieved by methods known to one skilled in the art such as by using fluidized bed equipment, rotating pharmaceutical pan, compression coating, spray coating or drenching. For example, wax may be applied onto the uncoated tablet core by spraying using any suitable spray equipment known in the art. In certain embodiments, the tablet core is coated by continuous spray methods.

Particularly, the step of directly coating the outer surface of the uncoated tablet core with a coating consisting of wax may comprise, transferring an uncoated tablet core into a coating pan, adding a wax powder to the coating pan, heating and rotating the coating pan.

Particularly, the step of directly coating the outer surface of the uncoated tablet core with a coating consisting of wax may comprise spraying molten wax onto the uncoated tablet core. Particularly, the step of directly coating the outer surface of the uncoated tablet core with a coating consisting of wax may comprise spraying molten wax onto the uncoated tablet core.

Particularly, the step of directly coating the outer surface of the uncoated tablet core comprises applying a wax coating having a total weight of from about 0.01% w/w to about 0.5% w/w based on the weight of the tablet core.

In preferred embodiments, the step of directly coating the outer surface of the uncoated tablet core with a coating consisting of wax may comprise, transferring an uncoated tablet core into a coating pan, adding a wax powder to the coating pan, heating and rotating the coating pan until the wax coating has a total weight of from about 0.01% w/w to about 0.5% w/w based on the weight of the tablet core.

The method for manufacturing may further comprise the step of packaging the coated tablets.

## General

The term "comprising" encompasses "including" e.g. a composition "comprising" X may include something additional e.g. X + Y. In some implementations, the term "comprising" refers to the inclusion of the indicated active agent, such as recited compounds, as well as inclusion of other active agents, and carriers, excipients, emollients, stabilizers, etc., known in the consumer health industry and generally recognized as safe (GRAS). Use of the transitional phrase "consisting essentially of" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461, 463 (CCPA 1976) (emphasis in the original); see also MPEP § 2111.03. Thus, the term "consisting essentially of" when used in a claim of this invention is not intended to be interpreted to be equivalent to "comprising". The term "consisting of" and variations thereof means including and limited to (for example, the specific recited constituent(s) or steps). In certain territories, the term "comprising an active ingredient consisting of" may be used in place of "consisting essentially". The term "about" in relation to a numerical value x is optional and means, for example, that the numerical value may comprise some variance around the stated number to allow for routine experimental fluctuation, measurement variance or to encompass minor deviations that may achieve substantially the same results as the stated number, such as x±10%, x±5%, x±4%, x±3%, x±2% or x±1%. The word "substantially" does not exclude "completely" e.g. a composition which is "substantially free" from Y may be completely free from Y. Where necessary, the word "substantially" may be omitted from the definition of the invention. All percentages and ratios used herein are by weight of total composition, unless otherwise indicated. For the avoidance of doubt, reference to percent by weight, "X% by weight", "X% w/w" and the like means by weight of the total composition, the amount of each ingredient being selected so that total ingredients in the composition sum to 100 percent by weight.

All references or patent applications cited within this patent specification are incorporated by reference herein. In order that this invention may be better understood, the following examples are set forth. These examples are for purposes of illustration only and are not to be construed as limiting the scope of the invention in any manner.

## Examples:

### Example 1:

A pharmaceutical composition comprising 500mg of Paracetamol and 25mg of diphenhydramine chloride as active pharmaceutical ingredients (APIs) was prepared.

| ACTIVES | FUNCTION | Unit Formula (mg/tablet) |
|---|---|---|
| **Paracetamol** | **Analgesic, Antipyretic** | 500 |
| **Diphenhydramine HCL** | **Sedating Effect** | 25 |
| **EXCIPIENTS** | | |
| Starch Maize | Disintegrant, Binder | 21.4 |
| Starch pregelatinised | Disintegrant, Binder, Diluent | 50 |
| Povidone | Binder | 2 |
| Potassium Sorbate | Preservative | 0.6 |
| Talc | Lubricant, Glidant, Anticaking agent | 15 |
| Stearic Acid | Lubricant, glidant, Emulsifying agent | 5 |
| Carnauba Wax | Tablet polishing | Trace |
| | **Target Tablet Weight** | 619mg |
| | **Tablet Dimensions** | 17.46mm x 7.14mm |

Diphenhydramine is an antihistamine used to relieve symptoms of allergy, hay fever, and the common cold. In combination with paracetamol, it is often also used to induce or improve sleep. Such combination products are currently marketed under the brand name Panadol® as "Panadol Night". However, these products have a film coating comprising a polymer, plasticizer and a pigment, which comes with the disadvantages described above.

**Manufacturing Method:**

Uncoated tablets were manufactured according to conventional processes well known to the person skilled in the art. The uncoated tablets were placed in a Vector Hi-Coater 170 coating pan. The air temperature was set to 40°C (Range 25 to 50°C), and the air heater is started. The tablets are allowed to warm with jogging intermittently, until the outlet temperature reaches 35°C. When the outlet temperature reached 35°C, the pan was rotated slowly (RPM < 2) until the temperature stabilised at > 35°C.

Parameter ranges were set as follows:

| PARAMETER | Coating Pan |
|---|---|
| Pan Size | 400 Kg (350 - 450 Kg) |
| Pan dP | 1 - 2 inches $H_2O$ (CP-1)<br>-25 to -50mm $H_2O$ (CP2/3) |
| Inlet air Volume* | 1900 - 2230m$^3$ /hr (CP-1)<br>3800 - 4100 m$^3$/hr (CP-2/3) |
| Outlet air Temp. | 20 - 50°C |
| Inlet air Temp.* | 25 - 50°C |
| Pan Speed** | 3 RPM |
| *Inlet air volume and temperature may be adjusted in order to achieve the desired exhaust temperature ranges.<br>**Pan speed RPM may be adjusted to achieve a good tablet mixing action inside the pan. | |

The inlet / exhaust air was turned off, and carnauba wax was added to the rolling tablet bed and the pan rotated for

3 minutes. Approximately 0.079kg of carnauba wax is used per 400kg of tablets. The inlet / exhaust air was turned on, following which the pan was rotated for a further 5 minutes. Inlet air was then turned off and the pan rotated for further 5 minutes. The tablets are continuously jogged until the exhaust air temperature has dropped to <40°C. The average weight of the polished tablets was then determined, and the polished tablets filled into double lined fibre board drums before being packed into blisters, containers, or other suitable packaging.

Film coating of Panadol Night tables takes approximately 3.5 hours to complete whilst the process described above was completed in approximately 20 minutes.

**Example 2:**

A pharmaceutical composition comprising 500mg of Paracetamol, 25mg of caffeine, and 5mg of phenylephrine HCl was prepared:

| **ACTIVES** | **FUNCTION** | **Unit Formula (mg/tablet)** |
|---|---|---|
| **Paracetamol** | **Analgesic, Antipyretic** | 500 |
| **Caffeine** | **Stimulating, Diuretic effect** | 25 |
| **Phenylephrine** | **Nasal Congestant** | 5 |
| **EXCIPIENTS** | | |
| Starch Maize | Disintegrant, Binder | 29.4 |
| Starch pregelatinised | Disintegrant, Binder, Diluent | 45 |
| Povidone | Binder | 5 |
| Potassium Sorbate | Preservative | 0.6 |
| Talc | Lubricant, Glidant, Anticaking agent | 12.5 |
| Stearic Acid | Lubricant, glidant, Emulsifying agent | 6.26 |
| Microcrystalline cellulose/Avicel | Glidant, diluent, disintegrant | 30 |
| Sodium Lauryl sulphate | Surf. Actant | 0.8 |
| Carnauba Wax | Polish | Trace |

Caffeine stimulates the central nervous system making cold & flu patients suffering from fatigue more alert. Phenylephrine HCl is used for the temporary relief of stuffy nose, sinus, and ear symptoms caused by the common cold, flu, allergies, or other breathing illnesses. Such combination products are currently marketed under the brand name Panadol® as "Panadol Cold & Flu" or under the brand Beechams® as "Beechams Flu Plus". However, the current Panadol Cold & Flu products have a film coating comprising a polymer, plasticizer and a pigment, which comes with the disadvantages described above.

The manufacturing process was the same as for Example 1.

**Example 3:**

A pharmaceutical composition comprising 250mg of Paracetamol and 125mg of Ibuprofen was prepared:

| Ingredient | Function | Reference to Standard | Unit formula | | |
|---|---|---|---|---|---|
| | | | mg/unit | % | % |
| **Tablet Core** | | | | | |
| Ibuprofen | Active | USP | 125.00 | 24.33 | 25.19 |
| Acetaminophen | Active | USP | 250.00 | 48.66 | 50.38 |
| Hypromellose (E15) | Binder | USP | 7.50 | 1.46 | 1.51 |
| Croscarmellose Sodium | Disintegrating Agent | NF | 33.25 | 6.47 | 6.70 |
| Colloidal Silicon Dioxide | Glidant | USP | 14.00 | 2.73 | 2.82 |

(continued)

| Ingredient | Function | Reference to Standard | Unit formula | | |
|---|---|---|---|---|---|
| | | | mg/unit | % | % |
| **Tablet Core** | | | | | |
| Pregelatinized Starch (corn) | Binder, Disintegrating Agent | NF | 60.00 | 11.68 | 12.09 |
| Purified Water | Processing Aid | USP | --[a] | --[a] | --[a] |
| Glyceryl Dibehenate | Lubricant | NF | 6.50 | 1.27 | 1.31 |
| **Wax Polish** | | | | | |
| Carnauba Wax #1 Yellow Powder | Polishing Agent | NF | 0.03 | 0.01 | |
| [a]removed during processing | | | | | |

Such combination products are currently marketed under the brand name Advil ® as "Advil Dual Action".

The manufacturing process was the same as for Example 1.

**Example 4** - **Comparative Slide Test**

In-vitro study was conducted to understand the effect of the removal of the conventional film coating and replacement by a wax polish directly applied on the uncoated tablet on the swallowability and flowability of the tablets. The studies involved the determination of the angle of slide.

The angle of slide is the angle at which the caplets overcome inertia and / or friction and move/ slide down an inclined plane without tumbling. The lower the angle of slide, the greater is the lubricity or slipperiness of the tablets. This further is co-related to the ease of swallowability and performance on packaging operation.

The study compared the commercially available Panadol Night tablets with the formulation according to Example 1. The commercially available Panadol Night product is based on the same uncoated tablet as the formulation of Example 1, but it is coated with an Opadry II Blue 33G20500 film coating, with each tablet comprising 19mg of coating, resulting in a total tablet weight of 638mg.

**Methodology**

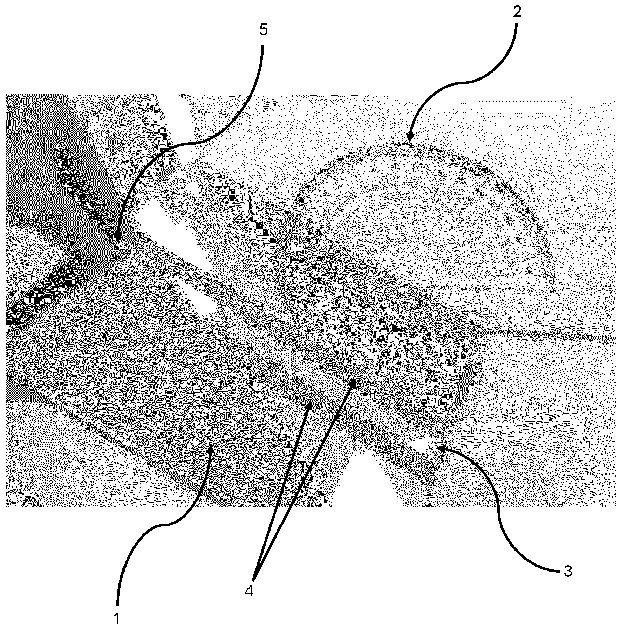

The experimental set-up is shown in Fig. 1.

1. A stainless-steel ramp was used to slide the tablets. A slide lane was established in the middle of the ramp using a paper ribbon.
2. 20 tablets from each sample were left at the top of ramp and observed for complete slide through the ramp.
3. The ramp angle was adjusted to an angle in which none of the 20 tablets from both samples slide through the ramp.
4. Ramp angle was gradually increased after each run, noting down the number of tablets from each sample that slid through the entire ramp. The way in which tablets slide (flat or rotating) was also noted.

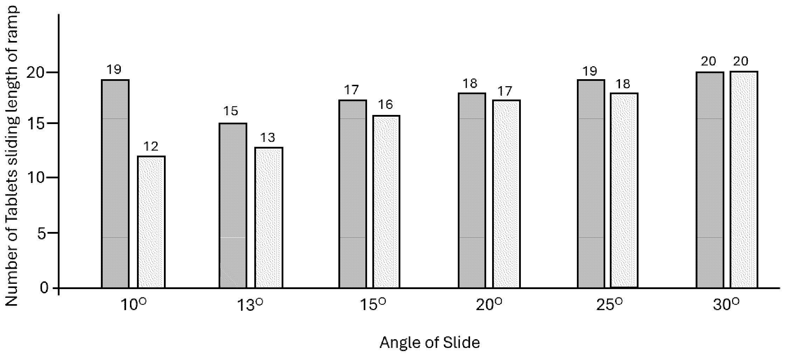

The results are shown in Figure 2.

Both formulations show the same angle of slide ranges, with no sliding at 5°, slide start at 10° and complete slide of all samples tested at 30°. The number of tablets sliding the entire ramp increases gradually with the increase of the ramp angle on both products. The tablets coated with wax showed only a slight decrease in the flowability with respect to the commercial product, more relevant at low angles. Tablets coated with wax only show almost the same trend to tumble when sliding through the ramp as the commercial product (data not shown).

The differences observed on the capacity to slide the ramp and the trend to rotate between both products, original and wax only, were not considered to be significant. Based on these tests, replacement of a film coating with a coating consisting of wax only would not affect a patient's perception of swallowability. However, in terms of processing, the use of a coating consisting of wax alone simplified processing whilst significantly reducing time needed to complete the process. Importantly, the new process represented a significant saving in terms of energy.

## Claims

1. A compressed pharmaceutical dosage form for oral administration comprising at least one active pharmaceutical ingredient (API) and one or more pharmaceutically acceptable diluents, excipients or carriers, wherein the pharmaceutical dosage form is a coated tablet, wherein the coating consists of wax and wherein the total weight of wax is from about 0.01% w/w to about 0.5% w/w based on the weight of the tablet core.

2. The compressed pharmaceutical dosage form of claim 1, wherein the at least one API is selected from the group consisting of diclofenac, ketoprofen, naproxen, sodium salicylate, acetylsalicylic acid, ibuprofen, dexibuprofen, paracetamol (acetaminophen), and combinations thereof.

3. The compressed pharmaceutical dosage form of claim 2 wherein the at least one API is selected from the group consisting of paracetamol, ibuprofen, dexibuprofen and combinations thereof.

4. The compressed pharmaceutical dosage form of claim 3 comprising from 200mg to 1000mg of paracetamol and/or from 80mg to 400mg of ibuprofen or equivalent ibuprofen salt.

5. The compressed pharmaceutical dosage form of claim 3 comprising from 200mg to 1000mg of paracetamol and/or from 40mg to 200mg of dexibuprofen or equivalent dexibuprofen salt.

6. The compressed pharmaceutical dosage form of claim 2 comprising from 125mg to 500mg of naproxen or equivalent naproxen salt.

7. The compressed pharmaceutical dosage form of claim 2 comprising from 25mg to 100mg of diclofenac or equivalent diclofenac salt.

8. The compressed pharmaceutical dosage form of any preceding claim wherein the wax is selected from the group consisting of carnauba wax, candelilla wax, beeswax (white or yellow), hard paraffin wax, cetyl palmitate, rice bran wax, and combinations thereof.

9. The compressed pharmaceutical dosage form of claim 8, wherein the wax is carnauba wax.

10. A method for manufacturing the compressed pharmaceutical dosage form of claims 1 to 9, the method comprising the steps of preparing an uncoated tablet core and directly coating the outer surface of the uncoated tablet core with a coating consisting of wax.

11. The method for manufacturing of claim 10, wherein the step of directly coating the outer surface of the uncoated tablet core with a coating consisting of wax comprises, transferring an uncoated tablet core into a coating pan, adding a wax powder to the coating pan, heating and rotating the coating pan.

12. The method for manufacturing of claim 11, wherein the step of directly coating the outer surface of the uncoated tablet core with a coating consisting of wax comprises spraying molten wax onto the tablet cores to provide coated tablets.

13. The method for manufacturing of claim 10, 11 or 12, further comprising the step of packaging the coated tablets.

14. The method for manufacturing of any one of claims 10 to 13, wherein the wax is carnauba wax.

5
2
1
4
3

Number of Tablets sliding length of ramp
0
5
10
15
20
Angle of Slide
10°
13°
15°
20°
25°
30°
19
12
15
13
17
16
18
17
19
18
20
20

Europäisches Patentamt
European Patent Office
Office européen des brevets

# EUROPEAN SEARCH REPORT

Application Number
EP 25 15 6471

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/009085 A2 (RANBAXY LAB LTD [IN]; MATHUR RAJEEV SHANKAR [IN] ET AL.) 29 January 2004 (2004-01-29) * claims 1-54; example 4 * | 1,8-14 | INV. A61K9/28 A61K31/135 A61K31/167 A61K31/192 A61K31/522 |
| X | WO 01/01991 A1 (MERCK & CO INC [US]; CHEN TZYY SHOW H [US] ET AL.) 11 January 2001 (2001-01-11) * abstract; claims 1-18; examples * | 1,8-14 | |
| X | CN 118 453 786 A (JIANGSU ZHONGYONG HONGRUI PHARMACEUTICAL CO LTD) 9 August 2024 (2024-08-09) * claims 7-9; examples 1-2 * | 1,8, 10-13 | |
| X | WO 2022/261610 A1 (SCHERER TECHNOLOGIES LLC R P [US]) 15 December 2022 (2022-12-15) * paragraph [0022]; claims 1-4 * | 1,2,8-14 | |
| X | US 4 256 747 A (TAUBER OSWALD ET AL) 17 March 1981 (1981-03-17) * example 3 * | 1,2,7-14 | TECHNICAL FIELDS SEARCHED (IPC) |
| X | CN 110 585 161 A (HENAN GENGXIANTANG PHARMACEUTICAL CO LTD) 20 December 2019 (2019-12-20) * claims 1-12; figure 1 * | 1,2,7-14 | A61K |

-/--

~~The present search report has been drawn up for all claims~~

3

| Place of search | Date of completion of the search | Examiner |
|---|---|---|
| The Hague | 23 July 2025 | Madalinska, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches
Patentamt
European
Patent Office
Office européen
des brevets

# EUROPEAN SEARCH REPORT

Application Number
EP 25 15 6471

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PATIL ARUN TRAMBAK ET AL: "Development and evaluation of a hot-melt coating technique for enteric coating", BRAZILIAN JOURNAL OF PHARMACEUTICAL SCIENCES, vol. 48, no. 1, 1 March 2012 (2012-03-01), pages 69-77, XP055859027, DOI: 10.1590/S1984-82502012000100008 Retrieved from the Internet: URL:https://www.researchgate.net/profile/Deepak-Khobragade/publication/262621875_Development_and_evaluation_of_a_hot-melt_coating_technique_for_enteric_coating/links/54af12380cf21670b3594f59/Development-and-evaluation-of-a-hot-melt-coating-technique-for-enteric-coating.pdf> * abstract; table I * ----- | 1,2,7-14 | |

TECHNICAL FIELDS SEARCHED (IPC)

~~The present search report has been drawn up for all claims~~

3

| Place of search | Date of completion of the search | Examiner |
|---|---|---|
| The Hague | 23 July 2025 | Madalinska, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

Europäisches Patentamt
European Patent Office
Office européen des brevets

Application Number

EP 25 15 6471

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  7(completely); 1, 2, 8-14(partially)

- [ ] The present supplementary European search report has been drawn up for those parts of the European patent application which relate to the invention first mentioned in the claims (Rule 164 (1) EPC).

Europäisches
Patentamt
European
Patent Office
Office européen
des brevets

# LACK OF UNITY OF INVENTION SHEET B

Application Number

EP 25 15 6471

The Search Division considers that the present European patent application does not comply with the requirements of unity of invention and relates to several inventions or groups of inventions, namely:

1. claims: 7(completely); 1, 2, 8-14(partially)

A compressed pharmaceutical dosage form for oral administration comprising at least one active pharmaceutical ingredient including diclofenac and one or more pharmaceutically acceptable diluents, excipients or carriers, wherein the pharmaceutical dosage form is a coated tablet, wherein the coating consists of wax and wherein the total weight of wax is from about 0.01% w/w to about 0.5% w/w based on the weight of the tablet core.

---

2. claims: 1, 2, 8-14(all partially)

A compressed pharmaceutical dosage form for oral administration comprising at least one active pharmaceutical ingredient including ketoprofen and one or more pharmaceutically acceptable diluents, excipients or carriers, wherein the pharmaceutical dosage form is a coated tablet, wherein the coating consists of wax and wherein the total weight of wax is from about 0.01% w/w to about 0.5% w/w based on the weight of the tablet core.

---

3. claims: 6(completely); 1, 2, 8-14(partially)

A compressed pharmaceutical dosage form for oral administration comprising at least one active pharmaceutical ingredient including naproxen and one or more pharmaceutically acceptable diluents, excipients or carriers, wherein the pharmaceutical dosage form is a coated tablet, wherein the coating consists of wax and wherein the total weight of wax is from about 0.01% w/w to about 0.5% w/w based on the weight of the tablet core.

---

4. claims: 1, 2, 8-14(all partially)

A compressed pharmaceutical dosage form for oral administration comprising at least one active pharmaceutical ingredient including sodium salicylate and one or more pharmaceutically acceptable diluents, excipients or carriers, wherein the pharmaceutical dosage form is a coated tablet, wherein the coating consists of wax and wherein the total weight of wax is from about 0.01% w/w to about 0.5% w/w based on the weight of the tablet core.

---

5. claims: 1, 2, 8-14(all partially)

A compressed pharmaceutical dosage form for oral

Europäisches
Patentamt
European
Patent Office
Office européen
des brevets

# LACK OF UNITY OF INVENTION SHEET B

Application Number
EP 25 15 6471

The Search Division considers that the present European patent application does not comply with the requirements of unity of invention and relates to several inventions or groups of inventions, namely:

administration comprising at least one active pharmaceutical ingredient including acetylsalicylic acid and one or more pharmaceutically acceptable diluents, excipients or carriers, wherein the pharmaceutical dosage form is a coated tablet, wherein the coating consists of wax and wherein the total weight of wax is from about 0.01% w/w to about 0.5% w/w based on the weight of the tablet core.

---

6. claims: 1-5, 8-14(all partially)

A compressed pharmaceutical dosage form for oral administration comprising at least one active pharmaceutical ingredient including ibuprofen or dexibuprofen and one or more pharmaceutically acceptable diluents, excipients or carriers, wherein the pharmaceutical dosage form is a coated tablet, wherein the coating consists of wax and wherein the total weight of wax is from about 0.01% w/w to about 0.5% w/w based on the weight of the tablet core.

---

7. claims: 1-5, 8-14(all partially)

A compressed pharmaceutical dosage form for oral administration comprising at least one active pharmaceutical ingredient including paracetamol and one or more pharmaceutically acceptable diluents, excipients or carriers, wherein the pharmaceutical dosage form is a coated tablet, wherein the coating consists of wax and wherein the total weight of wax is from about 0.01% w/w to about 0.5% w/w based on the weight of the tablet core.

---

# ANNEX TO THE EUROPEAN SEARCH REPORT ON EUROPEAN PATENT APPLICATION NO.

EP 25 15 6471

This annex lists the patent family members relating to the patent documents cited in the above-mentioned European search report. The members are as contained in the European Patent Office EDP file on
The European Patent Office is in no way liable for these particulars which are merely given for the purpose of information.

23-07-2025

| Patent document cited in search report | Publication date | Patent family member(s) | Publication date |
|---|---|---|---|
| WO 2004009085 A2 | 29-01-2004 | AU 2003249478 A1 | 09-02-2004 |
| | | BR 0312795 A | 10-05-2005 |
| | | CN 1681493 A | 12-10-2005 |
| | | EP 1524978 A2 | 27-04-2005 |
| | | US 2006233875 A1 | 19-10-2006 |
| | | WO 2004009085 A2 | 29-01-2004 |
| WO 0101991 A1 | 11-01-2001 | AR 024462 A1 | 02-10-2002 |
| | | AU 6055400 A | 22-01-2001 |
| | | CA 2378521 A1 | 11-01-2001 |
| | | EP 1196175 A1 | 17-04-2002 |
| | | HK 1041816 A1 | 26-07-2002 |
| | | JP 2003503460 A | 28-01-2003 |
| | | KR 20020015063 A | 27-02-2002 |
| | | NZ 515614 A | 30-01-2004 |
| | | US 2001036475 A1 | 01-11-2001 |
| | | US 2002187186 A1 | 12-12-2002 |
| | | WO 0101991 A1 | 11-01-2001 |
| CN 118453786 A | 09-08-2024 | NONE | |
| WO 2022261610 A1 | 15-12-2022 | AU 2022289906 A1 | 09-11-2023 |
| | | BR 112023022209 A2 | 06-02-2024 |
| | | CA 3219239 A1 | 15-12-2022 |
| | | CN 117440800 A | 23-01-2024 |
| | | CO 2023014571 A2 | 10-11-2023 |
| | | EP 4351526 A1 | 17-04-2024 |
| | | IL 308749 A | 01-01-2024 |
| | | JP 2024520160 A | 21-05-2024 |
| | | KR 20240019293 A | 14-02-2024 |
| | | US 2024285539 A1 | 29-08-2024 |
| | | WO 2022261610 A1 | 15-12-2022 |
| US 4256747 A | 17-03-1981 | AU 519763 B2 | 24-12-1981 |
| | | BE 864315 A | 24-08-1978 |
| | | CA 1107199 A | 18-08-1981 |
| | | CH 642548 A5 | 30-04-1984 |
| | | DE 2708520 A1 | 31-08-1978 |
| | | FR 2381526 A1 | 22-09-1978 |
| | | GB 1572779 A | 06-08-1980 |
| | | IE 46467 B1 | 15-06-1983 |
| | | IL 54129 A | 30-01-1981 |
| | | IT 1101769 B | 07-10-1985 |
| | | JP S6141896 B2 | 18-09-1986 |
| | | JP S53107424 A | 19-09-1978 |
| | | LU 79124 A1 | 25-05-1979 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 25 15 6471

This annex lists the patent family members relating to the patent documents cited in the above-mentioned European search report. The members are as contained in the European Patent Office EDP file on
The European Patent Office is in no way liable for these particulars which are merely given for the purpose of information.

23-07-2025

| Patent document cited in search report | Publication date | Patent family member(s) | Publication date |
|---|---|---|---|
| | | NL 7802023 A | 29-08-1978 |
| | | NZ 186563 A | 13-07-1981 |
| | | PH 15663 A | 11-03-1983 |
| | | PH 17554 A | 20-09-1984 |
| | | US 4154833 A | 15-05-1979 |
| | | US 4256747 A | 17-03-1981 |
| | | US 4292319 A | 29-09-1981 |
| | | ZA 781086 B | 31-10-1979 |
| CN 110585161 A | 20-12-2019 | NONE | |

EPO FORM P0459

For more details about this annex : see Official Journal of the European Patent Office, No. 12/82